(19) Europäisches Patentamt
European Patent Office
Office européen des brevets

(11) Publication number: **0 148 275 B1**

(12) # EUROPEAN PATENT SPECIFICATION

(45) Date of publication of patent specification: **02.09.92**

(51) Int. Cl.⁵: **C12Q 1/28**, G01N 33/48, G01N 33/82

(21) Application number: **84902364.3**

(22) Date of filing: **12.06.84**

(86) International application number: **PCT/JP84/00306**

(87) International publication number: **WO 84/04930 (20.12.84 84/30)**

(54) **PROCESS FOR DECOMPOSING ASCORBIC ACID.**

(30) Priority: **13.06.83 JP 105602/83**

(43) Date of publication of application: **17.07.85 Bulletin 85/29**

(45) Publication of the grant of the patent: **02.09.92 Bulletin 92/36**

(84) Designated Contracting States: **AT BE CH DE FR GB LI LU NL SE**

(56) References cited:
**JP-A- 5 540 909**
**JP-A- 5 918 000**
**JP-A- 5 920 853**
**JP-A-56 109 595**
**JP-B- 5 639 198**

**CLINICAL CHEMISTRY, vol. 28, no. 4, 1982, pages 578-588, Winston-Salem, US; R.H. WHITE-STEVENS: "Interference by ascorbic acid in test systems involving peroxidase. I. Reversible indicators and the effects of copper, iron, and mercury"**

(73) Proprietor: **WAKO PURE CHEMICAL INDUSTRIES, LTD.**
**10, Doshomachi-3-chome**
**Higashi-ku Osaka(JP)**

(72) Inventor: **YAMANISHI, Kazuhiko**
**17-10, Akatuka 3-chome Itabashi-ku**
**Tokyo 175(JP)**
Inventor: **HANADA, Toshiro**
**103, Minami Haitu 784, Oaza Minamiotuka**
**Kawagoe-shi Saitama 350(JP)**

(74) Representative: **Dr. Elisabeth Jung Dr. Jürgen Schirdewahn Dipl.-Ing. Claus Gernhardt**
**P.O. Box 40 14 68 Clemensstrasse 30**
**W-8000 München 40(DE)**

EP 0 148 275 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 82, no. 25, 23rd June 1975, page 204, abstract 166719j, Columbus, Ohio, US; K.A. SKOV et al.: "Kinetics of iron and copper catalysis of ascorbate oxidation", & BIOINORG. CHEM. 1975, 4(3), 199-213

CHEMICAL ABSTRACTS, vol. 82, no. 5, 3rd February 1975, page 115, abstract no. 26573g, Columbus, Ohio, US; D.B. DRATH et al.: "Bactericidal activity of metal-mediated peroxide-ascorbate systems", & INFECT. IMMUN. 1974, 10(5), 1077-83

CHEMICAL ABSTRACTS, vol. 70, no. 7, 17th February 1969, page 36, abstract no. 25919q, Columbus, Ohio, US; N. BAKARDZHIEVA: "Correlation of enzymic and nonenzymic oxidation of ascorbic acid in the presence of manganese, copper, and nickel", & DOKL. BOLG. AKAD. NAUK 1968, 21(10), 1113-16

## Description

TECHNICAL FIELD

The present invention relates to a novel method of decomposing ascorbic acid being present in a sample into dehydroascorbic acid and $H_2O$.

BACKGROUND TECHNIQUE

Ascorbic acid is a reducing substance publicly known as vitamin C, and it is well known that when it is coexistent with a component to be examined in a sample, for instance in a body fluid, a positive or negative measuring error is produced by the reducing property of ascorbic acid in the quantitative measurement of the respective intended component in the sample when utilizing for this purpose an oxidation-reduction reaction.

There have been reported as suitable ascorbic acid decomposing methods the following: (1) a method using ascorbate oxidase (Japanese Patent Publication No. 39,198/1981), (2) a method using iodic acid or a salt thereof, (3) a method using periodic acid or the like or a salt thereof (Japanese Patent Laid-Open No. 109,595/1981; Japanese Patent Laid-Open No. 151,358/1981; Japanese Patent Laid-Open No. 107,161/1981).

However, the above mentioned method (1) using ascorbate oxidase has the disadvantage that ascorbate oxidase is an enzyme and therefore a problem peculiar to the enzymes is observed, that is, this enzyme has poor thermal stability and poor storage stability. In addition, although it is strongly demanded by the user of such a method that a color is developed by a one step reaction using only a one liquid type reagent in which liquid the necessary reagents for carrying out the oxidation-reduction reaction are all present when the above method (1) is applied to this one step reaction, unless a large amount of ascorbate oxidase is used, the oxidase first acts upon a substrate which is the intended component in the measurement, so that the reaction producing hydrogen peroxide with the oxidase unfavorably proceeds, especially when ascorbic acid to be decomposed is still present. Using ascorbate oxidase in a large amount in order to avoid this unwanted side reaction is, however, uneconomical due to its high price.

Further, the method using iodic acid or the salt thereof and the method using periodic acid or the like or the salt thereof involve the problem that the enzymatic acticity of the oxidase is interfered with by the oxidizing action of the oxidizing agent in same cases. Moreover, even if the oxidase acts upon the substrate without its enzymatic activity being interfered with and thereby quantitatively generates hydrogen peroxide, there has not been developed up till now an oxidizable coloring reagent having the properties to form a measurable color at an appropriate pH-value, for leading the generated hydrogen peroxide to a coloring system is in coincidence with an appropriate pH at which ascorbic acid is decomposed by iodic acid or the salt thereof or by periodic acid or the salt thereof, so that the above method (2) or (3) cannot be applied to the one step reaction. Furthermore, when periodic acid or the salt thereof is used, a decomposing agent such as an alcohol or an aldehyde is required to decompose any excessive amount of periodic acid or of its salt, which has the consequence that the above method (3) cannot be applied in the one step reaction.

On the other hand, the reaction in which ascorbic acid is oxidized in the presence of divalent copper ion ($Cu^{2+}$ ion) to produce dehydroascorbic acid and $H_2O_2$ has been formerly known (see E.S. GUZMAN BARRON, R.H. DeMEIO, and FRIEDRICH KLEMPERER, J. Biol. Chem. 112, 625-640 (1936)). However, this is a reaction in which $H_2O_2$ is produced together with dehydroascorbic acid. In order to apply this decomposition reaction for a reaction in which $H_2O_2$ is produced by acting an oxidase upon a substrate and is measured quantitatively in order to analyze the intended component in a sample to be examined, the amounts of $H_2O_2$ produced in each of these reactions must be able to be distinguished and be detected separately, but it is needless to say that this is substantially impossible.

In order to solve this specific problem it has been suggested in "Clinical Chemistry" 28/4, pages 578-588 (1982) to use the mercuric ion instead of copper. But the inhibitory effect of the mercuric ion on the enzymatic activity must then be offset by using an increasing amount of the respective enzymes.

It is an object of the present invention to provide a method of decomposing ascorbic acid without producing hydrogen peroxide.

Further, it is another object of the present invention to provide a decomposition method which can effectively remove the effects of ascorbic acid contained in a reaction system used in the quantitative measurement of a component in a body fluid in which system $H_2O_2$ is produced by acting an oxidase upon a substrate, and the thus produced $H_2O_2$ is quantitatively measured.

It is still another object of the present invention to provide a method of decomposing ascorbic acid, in

which the decomposition of ascorbic acid and a reaction in which a sample to be examined is subjected to an enzyme reaction and both reactions can be carried out by one step, the enzyme reaction leading to a coloring system.

DISCLOSURE OF THE INVENTION

Unexpectedly, the present inventors have found during their studies of the decomposition reaction of ascorbic acid in the presence of copper ion that there exists a reaction in which ascorbic acid is oxidized in the presence of copper ion into dehydroascorbic acid and $H_2O$. Such a decomposition reaction needs the presence of peroxidase and one or more kinds of compounds selected from a group consisting of (1) 4-aminoantipyrine, (2) 3-methyl-2-benzothiazolinehydrazone, (3) 2,2'-azinobis(3-ethylbenzothiazoline-6-sulfonic acid), (4) triphenyl methane type leuco compounds, (5) phenol compounds, (6) aniline compounds, and (7) naphthol compounds in addition to the copper ion. Furthermore this method is applicable to the method of quantitativelymeasuring a component in a body fluid in which $H_2O_2$ is produced by acting an oxidase upon the substrate, and the thus produced $H_2O_2$ is measured in order to quantitatively analyze the intended component in the sample to be examined.

The present invention therefore comprises a method of decomposing ascorbic acid into dehydroascorbic acid and $H_2O$, which is characterized in that copper ion, peroxidase, and one or more kinds of compounds selected from the group consisting of the following (1) - (7):

(1) 4-aminoantipyrine (4-AAP)
(2) 3-methyl-2-benzothiazolinonehydrazone (MBTH)
(3) 2,2'-azinobis(3-ethylbenzothiazoline-6-sulfonic acid) (ABTS)
(4) triphenyl methane type leuco compounds
(5) phenol compounds
(6) aniline compounds
(7) naphthol compounds

are reacted with ascorbic acid.

The reaction of the invention proceeds according to the following formula:

$$\text{ascorbic acid} + 1/2\ O_2 \xrightarrow[\text{one or more kinds of compounds (1)-(7)}]{Cu^{2+},\ \text{peroxidase}} \text{dehydroascorbic acid} + H_2O$$

Taking into consideration the fact that up till now it was the general assumption that if $Cu^{2+}$ ion is present in the reaction system of the oxidation decomposition reaction of ascorbic acid, $H_2O_2$ is always produced together with dehydroascorbic acid, it is indeed unexpected and surprising that there does exist a reaction in which $H_2O$ is produced instead of $H_2O_2$.

Although it has been conventionally considered that the effective ion which decomposes ascorbic acid is the divalent copper ion, the inventors have discovered that $Cu^{2+}$ is present in the form of $Cu^{+}$ in the case that a reducing substance such as 4-aminoantipyrine or 3-methyl-2-benzothiazolinonehydrazone is coexistent and this copper ion decomposes ascorbic acid into the dehydroascorbic acid and $H_2O$. That is, the present inventors are the first to have found that the copper ion which decomposes ascorbic acid is not necessarily limited to the divalent copper ion and that the monovalent copper ion exhibits similar effect.

The present invention also comprises a method of quantitatively measuring an intended component in a body fluid, wherein a reducing substance is present, and of preventing any adverse influence of said substance, which is characterized in that copper ion, peroxidase, and one or more kinds of compounds selected from the group consisting of the following (1) - (7) are coexistent in a sample to be examined and containing said reducing substance, that the peroxidase is acting upon the intended component and produces $H_2O_2$, and that the thus produced $H_2O_2$ is quantitatively measured:

(1) 4-aminoantipyrine
(2) 3-methyl-2-benzothiazolinonehydrazone
(3) 2,2'-azinobis(3-ethylbenzothiazoline-6-sulfonic acid)
(4) triphenyl methane type leuco compounds
(5) phenol compounds

(6) aniline compounds
(7) naphthol compounds.

Therefore, when the method according to the present invention is applied to a reaction in which an oxidative substance such as $H_2O_2$ is measured in order to quantitatively analyze an intended component, the interference of the reducing substance such as ascorbic acid or bilirubin present in the reaction system can be effectively avoided, so that the intended component can be quantitatively measured with accuracy without being adversely affected by such interference.

If a triphenyl methane type leuco compound is used within the concept of the invention, it is preferably selected from the group consisting of leucomalachite green, leucocrystal violet, bis(p-diethylaminophenyl)-2-sulfophenyl methane, bis (p-diethylaminophenyl)-4-sulfopropoxyphenyl methane sodium salt and bis(p-diethylaminophenyl)-3,4-disulfopropoxyphenyl methane disodium salt (hereinafter referred to briefly as BSdiproPM).

The phenol compounds are suitably selected from the group comprising phenol, p-chlorophenol, 2,4-dichlorophenol, p-bromophenol, o-chlorophenol, and m-chlorophenol. The aniline compounds may be preferably selected from the group comprising aniline, N,N-dimethylaniline, N,N-diethylaniline, N,N-diethyl-m-toluidine, 3-methyl-N-ethyl-N-($\beta$-hydroxyethyl)-aniline, N-ethyl-N-(2-hydroxy-3-sulfopropyl)-m-toluidine, 3,5-dimethyl-N-ethyl-N-(2-hydroxy-3-sulfopropyl)aniline, and 3,5-dimethoxy-N-ethyl(2-hydroxy-3-sulfopropyl)-aniline.

Further, as suitable naphthol compounds mention may be made of 1-naphthol, 1-naphthol-2-sulfonic acid, 1-naphthol-2-carboxylicacid, 1-naphthol-8-sulfonic acid, 1-naphthol-3-sulfonic acid and 1-naphthol-5-sulfonic acid.

The decomposition reaction of the reducing substance, primarily ascorbic acid but in some cases also bilirubin, in the present invention is ordinarily carried out in a solution.

The concentration of $Cu^+$ or $Cu^{2+}$ in such a solution is set, for instance, at 0.001 - 1 m mol/l, and its pH is, for instance, at 6.0 - 8.5. As the buffer solution for maintaining the solution at such a pH, use may be made of, for instance, phosphate buffer solution, tris(hydroxymethyl)aminomethane salt buffer solution, Good buffer solution and the like which are widely employed for such a purpose. As the preferred example thereof, mention may be made of 0.001 - 2 M phosphate buffer solution.

The compound which is the source of the $Cu^+$ or $Cu^{2+}$ ion in such a solution may be a water-soluble copper compound, for example a water-soluble inorganic copper salt such as copper sulfate, cupric chloride, cuprous chloride, copper nitrite, cupric bromide, cuprous bromide, cupric phosphate, or a water-soluble organic copper salt such as copper tartrate, copper citrate, and copper acetate.

The concentration of peroxidase used in the present invention is ordinarily 20 - 5,000 U/dl, and preferably 50 - 2,000 U/dl.

The concentration of 4-aminoantipyrine (4-AAP) is ordinarily 0.001 - 0.05%, preferably 0.003 - 0.03%.

The concentration of 3-methyl-2-benzothiazolinonehydrazone (MBTH) is ordinarily 0.0005 - 0.2%, preferably 0.001 - 0.05%.

The concentration of 2,2'-azinobis(3-ethylbenzothiazoline-6-sulfonic acid) (ABTS) is ordinarily 0.002 - 0.4%, preferably 0.02 - 0.2%.

The concentration of the triphenyl methane type leuco compound is ordinarily 0.005 - 0.5 m mol/l, preferably 0.03 - 0.3 m mol/l.

The concentration of the phenol compound, the aniline compound or the naphthol compound is ordinarily 0.01 - 0.5%, preferably 0.03 - 0.3%.

The method according to the present invention can also be carried out by using a test paper in which all reagents are present in the dried state in an absorbing carrier or film.

When the method according to the present invention is performed as a pretreatment prior to the measurement of a component in a body fluid, ascorbic acid and bilirubin are rapidly oxidized, and their interfering action disappears.

A measuring method which is particularly effective when the method according to the present invention is applied is a method in which a sample to be examined is a body fluid and the intended component to be quantitatively measured is a body fluid component. A typical example for such a measuring method is an enzyme reaction in which the intended component to be quantitatively measured is either a substrate or an enzyme, and the enzyme when acting upon the substrate causes $H_2O_2$ to be produced. As examples of such a reaction, mention can be made of systems in which the substrate is glucose, cholesterol, glycerol, glycerol phosphate, choline, acylCoA, pyruvic acid, uric acid, xanthine or lactice acid, and the oxidase acting upon these substrates is glucose oxidase, cholesterol oxidase, glycerol oxidase, glycerol phosphate oxidase, choline oxidase, acylCoA oxidase, pyruvic acid oxidase, uricase, xanthine oxidase or lactic acid oxidase.

When the $H_2O_2$ produced in such a reaction is measured in order to analyze the intended component, it is common practice that an oxidizable coloring reagent is used , which is colored by being oxidized in the presence of $H_2O_2$ and peroxidase, and the color of the reagent developed by the $H_2O_2$ is measured in order to quantitatively analyze the intended component.

As the oxidizable coloring reagent used for this purpose mention may be made of a combined reagent of 4-aminoantipyrine (4-AAP) and a phenol compound, a combined reagent of 4-aminoantipyrine (4-AAP) and an aniline compound, a combined reagent of 4-aminoantipyrine (4-AAP) and a naphthol compound, a combined reagent of 3-methyl-2-benzothiazolinonehydrazone (MBTH) and an aniline compound, 2,2'-azinobis(3-ethylbenzothiazoline-6-sulfonic acid) (ABTS) or a triphenyl methane type leuco compound, which are identical with the reagents necessary for decomposing ascorbic acid without producing $H_2O_2$ according to the present invention. Therefore as specific examples of the triphenyl methane type leuco compound, the phenol compound, the aniline compound and the naphthol compound there can be mentioned those comprised in the group referred to above.

Examples of the quantitatively measuring reagents used in the method of quantitatively measuring a body fluid component in which $H_2O_2$ is produced by acting an oxidase upon a substrate are the following:

(1) In case of the measurement of glucose, a reagent which contains peroxidase, glucose oxidase, 4-aminoantipyrine, phenol or N,N-diethylxylidine and a buffer.

(2) In case of the measurement of uric acid, a reagent which contains peroxidase, uricase, N-ethyl-N-($\beta$-hydroxyethyl)-m-toluidine, 4-aminoantipyrine and a buffer.

(3) In case of the measurement of cholesterol, a reagent which contains peroxidase, cholesterol ester hydrolase, cholesterol oxidase, 4-aminoantipyrine, phenol or N-ethyl-N-($\beta$-hydroxyethyl)-m-toluidine and a buffer.

(4) In case of the measurement of a triglyceride, a reagent which contains peroxidase, lipoprotein lipase, glycerol kinase, glycerol-3-phosphate oxidase, 4-aminoantipyrine, p-chlorophenol or N-ethyl-N-($\beta$-hydroxyethyl)-m-toluidine and a buffer.

(5) In case of the measurement of a phospholipid, a reagent which contains peroxidase, phospholipase D, choline oxidase, 4-aminoantipyrine, phenol or N-ethyl-N-($\beta$-hydroxyethyl)-m-toluidine and a buffer.

(6) In case of the measurement of acylCoA, a reagent which contains peroxidase, acylCoA oxidase, 4-aminoantipyrine, p-chlorophenol or N-ethyl-N-($\beta$-hydroxyethyl)-m-toluidine and a buffer.

(7) In case of the measurement of pyruvic acid, a reagent which contains peroxidase, pyruvate oxidase, flavin adenine dinucleotide, thiamine pyrophosphate, 4-aminoantipyrine, p-chlorophenol or N-ethyl-N-($\beta$-hydroxyethyl)-m-toluidine and a buffer.

(8) In case of the measurement of glycerol, a reagent which contains peroxidase, glycerol oxidase, 4-aminoantipyrine, p-chlorophenol or N-ethyl-N-($\beta$-hydroxyethyl)-m-toluidine and a buffer.

(9) In case of the measurement of choline, a reagent which contains peroxidase, choline oxidase, 4-aminoantipyrine, phenol or N-ethyl-N-($\beta$-hydroxyethyl)-m-toluidine and a buffer.

(10) In case of the measurement of glycerol-3-phosphate, a reagent which contains peroxidase, glycerol-3-phosphate oxidase, 4-aminoantipyrine, p-chlorophenol or N-ethyl-N-($\beta$-hydroxyethyl)-m-toluidine and a buffer.

(11) In case of the measurement of uric acid, a reagent which contains peroxidase, uricase, 2,2'-azinobis-(3-ethylbenzothiazoline-6-sulfonic acid) and a buffer.

When the ascorbic acid-decomposing method according to the present invention is applied to the method of quantitatively measuring the body fluid component by using the above-mentioned quantitatively measuring reagents, it is possible to effectively avoid the interference of a reducing substance such as ascorbic acid or bilirubin which are coexisting in the reaction system in which the oxidizing substance or a reducing substance is measured in order to quantitatively analyzing the intended component. In the case that the reaction is done in one step using one liquid type reagent in which the necessary reagents are all present in a single liquid or the reagents are gathered into a single liquid in the measuring system, such an embodiment being most required when using the oxidation-reduction reaction (redox reaction), the intended component can be quantitatively measured with accuracy without being adversely affected by such an interference of reducing substances.

For instance, in order to quantitatively measure free cholesterol in serum, when $CuSO_4 \cdot 5H_2O$, 4-AAP, a phenol compound, an aniline compound or a naphthol compound, peroxidase, cholesterol oxidase and a surface active agent are added to give concentrations of 0.001 - 1 m mol/ℓ, 0.003 - 0.03%, 0.03 - 0.3%, 50 - 2,000 U/dℓ, 5 - 100 U/dℓ and 0.05 - 0.2% respectively, and a buffer solution dissolving these compounds is used as a measuring reagent, the interference of the reducing substance such as ascorbic acid and bilirubin present in the serum can be effectively avoided thereby measure the making it possible to measure the value of free cholesterol with more accuracy.

BRIEF DESCRIPTION OF THE DRAWINGS

Fig. 1 is a calibration curve obtained in Example 7, in which absorbances (OD) are plotted along an ordinate obtained with respect to the concentration (mg/dl) of total cholesterol plotted on an abscissa and the plotted points are connected by a straight line;
Fig. 2 is a calibration curve obtained in Example 8, in which absorbances (OD) obtained with respect to the concentration (mg/dℓ) of glucose are plotted along in an identical manner as in Fig.1.

BEST MODE FOR PRACTICING THE INVENTION

Ascorbic Acid Decomposition:

Example 1:

(Measuring reagent)

(1) First reagent:

$CuSO_4 \cdot 5H_2O$, peroxidase (POD), phenol, 1-naphthol-2-sulfonic acid, and 4-AAP are used either singly or in a combination of more than one of the components in 0.05 M phosphate buffer solution (pH = 7.0) in the composition shown in following Table 1,with the proviso that $CuSO_4 \cdot 5H_2O$, POD, phenol, and 1-naphthol-2-sulfonic acid were adjusted to be at concentrations of 0.003%, 300 U/dℓ, 0.1%, and 0.01% respectively.

(2) Second reagent (coloring reagent):

A coloring reagent solution was prepared by dissolving POD, phenol, and 4-AAP in 0.05 M phosphate buffer solution (pH = 7.0) to give concentrations of 300 U/dℓ, 0.1%, and 0.01% respectively.

In the embodiment where the reagent solution containing 1-naphthol-2-sulfonic-acid was used as the first reagent solution, the reagent solution in which POD, 1-naphthol-2-sulfonic acid, and 4-AAP are dissolved in 0.05 M phosphate buffer solution (pH = 7.0) to give concentrations of 300 U/dℓ, 0.1%, and 0.01% respectively was used as the second reagent solution (coloring reagent solution).

(Measuring method)

After 50 μℓ of an ascorbic acid solution (100 mg/dℓ) were sampled, 2 mℓ of the first reagent solution were added thereto, and the mixture was incubated at 37°C for 3 minutes. Then, 2 mℓ of the second reagent solution (coloring reagent solution) was added thereto, and the mixture was incubated at 37°C for 5 minutes. The absorbance at a wavelength of 505 nm was measured with reference to a reagent blank as a control. Results are shown in Table 1(examples *) are according to the invention, the other examples being used as reference).

Table 1

| Composition in the first reagent solution | Absorbance (OD) |
|---|---|
| $CuSO_4$ | 0.205 |
| $CuSO_4$, POD | 0.164 |
| $CuSO_4$, POD, 4-AAP | 0.000 *) |
| $CuSO_4$, POD, phenol | 0.001 *) |
| $CuSO_4$, POD, 1-naphthol-2-sulfonic acid | 0.001 *) |
| $CuSO_4$, POD, phenol, 4-AAP | 0.001 *) |
| $CuSO_4$, POD, 1-naphthol-2-sulfonic acid, 4-AAP | 0.001 *) |
| $CuSO_4$, 4-AAP | 0.206 |
| $CuSO_4$, phenol | 0.203 |
| $CuSO_4$, 1-naphthol-2-sulfonic acid | 0.202 |
| Note: When $H_2O_2$ is produced through decomposition of ascorbic acid, the compounds 4-AAP and phenol or 4-AAP and 1-naphthol-2-sulfonic acid present in the systems are oxidized with the thus produced $H_2O_2$ and an absorption appears in the vicinity of a wavelength of 505 nm. | |

It is seen from these experiments that when 4-AAP, phenol, 1-naphthol-2-sulfonic acid, phenol and 4-AAP, or 4-AAP and 1-naphthol-2-sulfonic acid are coexistent with copper ion and POD, no production of $H_2O_2$ is observed. In the embodiments where any one of the compounds 4-AAP, phenol and 1-naphthol-2-sulfonic acid is coexistent in the first reagent solution with copper ion and POD, even if $H_2O_2$ would be produced, such $H_2O_2$ is removed in situ by POD and 4-AAP, phenol or 1-naphthol-2-sulfonic acid, and thus no $H_2O_2$ can be detected from the appearance of the sample (since even oxidation produces no great change when viewed from an external appearance in the case of 4-AAP alone, phenol alone, or 1-naphthol-2-sulfonic acid alone; whether or not $H_2O_2$ is produced therefore cannot be confirmed in the above experiment).

Taking this into account, an experiment was next made to confirm that even in these cases , ascorbic acid is decomposed without being accompanied by the production of $H_2O_2$.

Experiment 2:

(Measuring reagent)

(1) First reagent solution:

$CuSO_4 \cdot 5H_2O$, POD, and 4-AAP (or phenol or 1-naphthol-2-sulfonic acid) were dissolved in 0.05 M phosphate buffer solution (pH = 7.5) to be at concentrations of 0.003%, 300 U/dℓ, and 0.017% respectively (0.01% in the case of phenol or 1-naphthol-2-sulfonic acid).

(2) Second reagent solution (coloring reagent solution):

$NaIO_4$ and phenol(or 4-AAP) were dissolved in 0.05 M phosphate buffer solution (pH = 7.5) to give concentrations of 20 mg/dℓ and 0.1% respectively (0.01% in the case of 4-AAP).

When the reagent solution containing 4-AAP was used as the first reagent solution, the reagent solution containing phenol was used as the second reagent solution, while when the reagent solution containing phenol or 1-naphthol-2-sulfonic acid was used as the first reagent solution, the reagent solution containing 4-AAP was used as the second reagent solution.

(Measuring method)

As samples, a solution in which ascorbic acid was dissolved in 0.05 M phosphate buffer solution (pH = 7.5) at a concentration of 0 mg/dℓ, 100 mg/dℓ, or 200 mg/dℓ (11 m mol/ℓ) or a solution in which $H_2O_2$ was

dissolved in 0.05 M phosphate buffer solution at a concentration of 0 m mol/ℓ, 5 m mol/ℓ, or 10 m mol/ℓ were used.

To 100 μℓ of the sample were added 2 mℓ of the first reagent liquid , and this mixture was incubated at 37°C for 10 minutes. To 100 μℓ of the resulting mixed solution were added 3 mℓ of the second reagent solution (coloring reagent solution) and this mixture was incubated at 37°C for 5 minutes. Thereafter, the absorbance at a wavelength of 505 nm was measured with reference to a reagent blank as a control. Measured results are shown in Table 2.

Table 2

| Sample | Ascorbic acid | | | Sample | $H_2O_2$ | | |
|---|---|---|---|---|---|---|---|
| * / Sample concentration | 4-AAP | Phenol | 1-Naphthol-2-sulfonic acid | * / Sample concentration | 4-AAP | Phenol | 1-Naphthol-2-sulfonic acid |
| 0 (mg/dℓ) | 0.278 | 0.361 | 0.360 | 0 (m mol/ℓ) | 0.282 | 0.370 | 0.368 |
| 100 | 0.281 | 0.352 | 0.358 | 5 | 0.197 | 0.190 | 0.187 |
| 200 (11 m mol/ℓ) | 0.278 | 0.359 | 0.358 | 10 | 0.123 | 0.092 | 0.090 |

* : component other than copper ion and POD in the first reagent solution

From the above results it can be concluded that since the coexisting 4-AAP, phenol, and 1-naphthol-2-sulfonic acid undergo no change at all in the decomposition of ascorbic acid with copper ion, no $H_2O_2$ is produced in this reaction system. On the other hand, when $H_2O_2$ is used as the sample in a substantially equal mole to the ascorbic acid, 4-AAP, phenol, and 1-naphthol-2-sulfonic acid are apparently oxidized by the $H_2O_2$. In the 4-AAP coexisting system, red color developed, while in the phenol system and in the naphthol system a yellowish brown color developed. When the remaining concentration of the $H_2O_2$ in the reaction system is measured, it is recognized that the $H_2O_2$ has been apparently consumed.

Example 3:

(Measuring reagent)

(1) First reagent solution:

$CuSO_4 \cdot 5H_2O$, POD, 3-methyl-N-ethyl-N-(β-hydroxyethyl)-aniline (MEHA), and 3-methyl-2-benzothiazolinonehydrazone (MBTH) were either singly or in a combination of more than one kind of component dissolved in 0.05 M phosphate buffer solution (pH = 7.0) in the composition shown in the following Table 3, with the proviso that $CuSO_4 \cdot 5H_2O$, POD, MEHA and MBTH were adjusted to be at concentrations of 0.003%, 300 U/dℓ, 0.05% and 0.03% respectively.

(2) Second reagent solution (coloring reagent solution):

When the reagent solution containing MEHA was used as the first reagent solution, a solution in which POD and 4-AAP were dissolved in 0.05 M phosphate buffer solution (pH = 7.0) to give concentrations of 300 U/dℓ and 0.01% respectively was used as the second reagent solution (coloring reagent solution), while when the reagent solution containing MBTH was used as the first reagent solution, a solution in which POD and MEHA were dissolved in 0.05 M phosphate buffer solution (pH = 7.0) to give concentrations of 300

U/dℓ and 0.02% respectively was used as the second reagent solution (coloring reagent solution). When neither MEHA nor MBTH were contained in the first reagent solution, a solution in which POD, MEHA and 4-AAP were dissolved in 0.05 M phosphate buffer solution (pH = 7.0) to give concentrations of 300 U/dℓ, 0.05% and 0.01% respectively was used as the second reagent solution (coloring reagent solution).

(Measuring method)

50 μℓ of an ascorbic acid solution (100 mg/dℓ) was sampled, 2 mℓ of the first reagent solution were added thereto,and the mixture was incubated at 37°C for 5 minutes. Then, 2 mℓ of the second reagent solution were added, and the mixture was incubated at 37°C for 5 minutes. Thereafter,the absorbance at a wavelength of 550 nm was measured with reference to a reagent blank as a control. Measured results are shown in Table 3 ( experiments *) are according to the invention, the other experiments being used as reference).

Table 3

| Composition in the first reagent solution | Absorbance (OD) |
|---|---|
| $CuSO_4$ | 0.434 |
| $CuSO_4$, POD | 0.314 |
| $CuSO_4$, POD, MEHA *) | -0.001 |
| $CuSO_4$, POD, MBTH *) | 0.004 |
| $CuSO_4$, MEHA | 0.416 |
| $CuSO_4$, MBTH | 0.073 |

It seems from this experiment that when MEHA or MBTH besides copper ion and POD is coexistent in the first reagent solution, no $H_2O_2$ is produced when viewed from the external appearance. However, whether or not ascorbic acid is actually decomposed without being accompanied by the production of $H_2O_2$ can not be known from this experiment, and therefore, the following experiment was made to confirm this.

Example 4:

(Measuring reagent)

(1) First reagent solution:

$CuSO_4 \cdot 5H_2O$, POD and MEHA (or MBTH) were dissolved in 0.05 M phosphate buffer solution (pH = 7.5) to give concentrations of 0.003%, 300 U/dℓ and 0.019% (0.025% in the case of MBTH).

(2) Second reagent solution (coloring reagent solution):

$NaIO_4$, and 4-AAP (or MEHA) were dissolved in 0.05 M phosphate buffer solution (pH = 7.5) to give concentrations of 20 mg/dℓ and 0.01% respectively (0.01% in the case of MEHA).

When the reagent solution containing MEHA was used as the first reagent solution, the reagent solution containing 4-AAP was used as the second reagent solution, while when the reagent solution containing MBTH was used as the first reagent solution, the reagent solution containing MEHA was used as the second reagent solution.

(Measuring method)

As samples, a solution in which ascorbic acid was dissolved in 0.05 M phosphate buffer solution (pH = 7.5) to give concentration of 0 mg/dℓ, 100 mg/dℓ or 200 mg/dℓ (11 m mol/ℓ), or a solution in which $H_2O_2$ was dissolved in 0.05 M phosphate buffer solution to give a concentration of 0 m mol/ℓ, 5 m mol/ℓ or 10 m mol/ℓ was used.

To 100 μℓ of each sample were added 2 mℓ of the first reagent solution, and the mixture was

incubated at 37° C for 10 minutes. To 100 μℓ of the resulting mixed solution were added 3 mℓ of the second reagent solution, and this mixture was incubated at 37° C for 5 minutes. Thereafter,the absorbance at a wavelength of 550 nm was measured with reference to a reagent blank as a control. Measured results are shown in Table 4.

Table 4

| Sample | Ascorbic acid | | Sample | $H_2O_2$ | |
|---|---|---|---|---|---|
| Sample* / Concentration | MEHA | MBTH | Sample* / Concentration | MEHA | MBTH |
| 0 (mg/dℓ) | 0.900 | 0.539 | 0 (m mol/ℓ) | 0.920 | 0.554 |
| 100 | 0.896 | 0.538 | 5 | 0.472 | 0.285 |
| 200 (11 m mol/ℓ) | 0.898 | 0.536 | 10 | 0.230 | 0.138 |

*: Component other than copper ion and POD in the first reagent solution

From the above, it can be concluded that since the coexisting MEHA and MBTH are not changed at all in the decomposition of ascorbic acid with copper ion, no $H_2O_2$ is produced in this reaction system. On the other hand, when $H_2O_2$ in a substantially equal mol concentration to the ascorbic acid is used as a sample, MEHA and MBTH are apparently oxidized with the $H_2O_2$, and it can be verified by the measurement of the remaining $H_2O_2$ that the $H_2O_2$ is actually consumed.

Example 5:

(Measuring reagent)

(a) A solution in which $CuSO_4 \cdot 5H_2O$, POD and ABTS were dissolved in 0.05 M phosphate buffer solution (pH = 7.0) to give concentrations of 0.03%, 300 U/dℓ and 0.1% respectively.

(b) A solution in which $CuSO_4 \cdot 5H_2O$, POD and bis(p-diethylaminophenyl)-3,4-disulfopropoxyphenyl methane disodium salt (hereinafter abbreviated as BSdiproPM) were dissolved in 0.05 M phosphate buffer solution (pH = 7.0) to give concentrations of 0.003%, 300 U/dℓ, and 0.05 mM respectively.

(Measuring method)

50 μℓ of an ascorbic acid solution (100 mg/dℓ)were sampled, the measuring reagent solution (a) or (b) was added thereto, and the mixture was incubated at 37° C for 3 minutes. Then, the absorbance at a wavelength of 660 nm or 620 nm was measured with reference to a reagent blank as a control. Measured results area shown in Table 5.

Table 5

| Composition in the measuring reagent solution | Absorbance (OD) |
|---|---|
| $CuSO_4$, POD, ABTS | 0.000 |
| $CuSO_4$, POD, BSdipropM | 0.000 |

It is seen from this experiment that when the copper ion, POD and ABTS are coexistent, and when the copper ion, POD and triphenyl methane type leuco coloring matter are coexistent, ascorbic acid is decomposed without being accompanied by the production of $H_2O_2$.

In order to confirm whether the copper ion is present in the divalent or monovalent form in the reaction system of the present invention, an experiment was made by using bathocuproine disulfonic acid, 2 Na salt as a coloring reagent of $Cu^+$.

Example 6:

(Measuring sample):

$CuSO_4 \cdot H_2O$, POD, phenol, 1-naphthol-2-sulforic acid, 4-AAP, ABTS, BsdipropM, and MBTH were singly or in a combination of more than one kind of compound dissolved in 0.05 M phosphate (pH = 7.0), with the proviso that $CuSO_4 \cdot 5H_2O$, POD, phenol, 1-naphthol-2-sulfonic acid, 4-AAP, ABTS, BsdiproPM and MBTH were adjusted to be at concentrations of 0.03%, 300 U/dℓ, 0.1%, 0.1%, 0.01%, 0.1%, 0.1 m mol/ℓ, and 0.1% respectively.

(Coloring reagent solution)

Bathocuproine disulfonic acid, 2 Na salt was dissolved in 0.5 M phosphate buffer solution (pH = 4.4) to give a concentration of 10 mg/dℓ.

(Measuring method)

To 0.5 mℓ of a measuring sample of each of the various compositions of Table 6 were added 2 ml of a coloring reagent solution, and the absorbance at a wavelength of 480 nm was measured, from which the concentration of $Cu^+$ in the measuring sample was calculated. Further, 20 mg of ascorbic acid were added to this reaction solution, and the absorbance at a wavelength of 480 nm was measured again, from which the concentration of $Cu^+$ in the measuring sample was calculated. Measured results are shown in Table 6.

Table 6

| Composition in the measuring sample | $Cu^+$ concentration (μg/dℓ) | |
|---|---|---|
| | Before Ascorbic acid addition | After Ascorbic acid addition |
| $CuSO_4$ | 11 | 756 |
| $CuSO_4$, POD | 7 | 776 |
| $CuSO_4$, POD, 4-AAP | 746 | 734 |

| | | |
|---|---|---|
| $CuSO_4$, POD, phenol | 5 | 753 |
| $CuSO_4$, POD, 4-AAP, phenol | 738 | 762 |
| $CuSO_4$, 4-AAP | 763 | 781 |
| $CuSO_4$, phenol | 6 | 758 |
| $CuSO_4$, 4-AAP, phenol | 752 | 753 |
| $CuSO_4$, 1-naphthol-2-sulfonic acid | 5 | 760 |
| $CuSO_4$, ABTS | — * | 762 |
| $CuSO_4$, BSdiproPM | 12 | 767 |
| $CuSO_4$, MBTH | 743 | 758 |

* Since the reaction solution becomes yellowish green, distinction is impossible. However, when 4-AAP was added to this system, it became yellowish brown. Thus, it was confirmed that $Cu^{2+}$ had changed to $Cu^{+}$, which had reacted with bathocuproine disulfonic acid, 2 Na salt. Accordingly, it is seen that $Cu^{+}$ is not produced in the case of ABTS and $Cu^{2+}$.

It is seen from the above experiment that when phenol, 1-naphthol-2-sulfonic acid, ABTS or BsdiproPM is coexistent, divalent Cu ion is present as it is, while when 4-AAP or MBTH is coexistent, it is present as a monovalent copper ion.

Decomposition of ascorbic acid or bilirubin in a measuring system of body fluid component

Example 7: Measurement of total cholesterol in serum

(Measuring reagent)

Copper sulfate, phenol, 4-aminoantipyrine, peroxidase (POD), cholesterol ester hydrolase, cholesterol oxidase and Rochelle salt and Triton X-100 are dissolved in 0.05 M phosphate buffer solution (pH = 7.5) to give concentrations of 0.12 m mol/ℓ, 0.1%, 0.01%, 600 U/dℓ, 30 U/dℓ, 15 U/dℓ, 0.5%, and 0.1% respectively.

(Measuring method)

To 100 ml samples of serum ascorbic acid was added in an amount of 0, 5, 10, 20, 30, 60, 80 and 100 mg/dl and these solutions were used as samples.

To 20 µl of each sample solution were added 3 ml of the measuring reagent, and the mixture was incubated at 37° C for 10 minutes. Thereafter, the absorbance at 505 nm was measured with reference to a reagent blank as a control.

The concentration of the total cholesterol in the sample was calculated from a separately prepared calibration curve (Fig.1). Measured results are shown in Table 7.

Comparative Example 1:

(Measuring reagent)

The same as the measuring reagent in Example 7 except that $CuSO_4 \cdot 5H_2O$ was excluded therefrom .

(Measuring method)

The total cholesterol was measured by using the same sample solution as in Example 7 according to the measuring method in Example 7. Measured results are shown in Table 7.

Reference Example 1:

(Measuring reagent)

The same as the measuring reagent in Example 7 except that ascorbic oxidase (AOD) was added to give a concentration of 100 U/dℓ instead of $CuSO_4 \cdot 5H_2O$.

(Measuring method)

The total cholesterol was measured by using the same sample solution as in Example 7 according to the measuring method in Example 7. Measured results are shown in Table 7.

Table 7

| Added amount of ascorbic acid (mg/dℓ) | Total cholesterol concentration (mg/dℓ) | | |
|---|---|---|---|
| | Example 7 | Comparative Example 1 | Reference Example 1 |
| 0 | 243 (100) | 240 (100) | 241 (100) |
| 5 | 245 (100.8) | 225 ( 93.8) | 241 (100) |
| 10 | 240 ( 98.8) | 210 ( 87.5) | 240 ( 99.6) |
| 20 | 241 ( 99.2) | 187 ( 77.9) | 235 ( 97.5) |
| 30 | 233 ( 95.9) | 163 ( 67.9) | 230 ( 95.4) |
| 60 | 178 ( 73.3) | 88 ( 36.7) | 180 ( 74.7) |
| 80 | 158 ( 65.0) | 51 ( 21.3) | 149 ( 61.8) |
| 100 | 140 ( 57.6) | 6 ( 2.5) | 139 ( 57.7) |
| Note: Figures in parentheses mean recovery rates (%) of cholesterol | | | |

As shown in Table 7, when up to 20 mg/dℓ of ascorbic acid are coexistent in Example 7 in which the copper ion, POD, phenol, and 4-AAP are also coexistent, no influence is observed upon the values of the total cholesterol, while when even only 5 mg/dℓ of ascorbic acid are coexistent in Comparative Example 1, a negative influence appears.

Further, as shown in Table 7, it is understood that the effect of removing the interference of ascorbic acid according to the method of the present invention is substantially equal to that of decomposing it with AOD.

Example 8: Measurement of glucose in serum

(Measuring reagent)

Copper sulfate, phenol, 4-aminoantipyrine, peroxidase, glucose oxidase, mutarotase, and Rochelle salt were dissolved in 0.05 M phosphate buffer solution (pH = 7.0) to give concentrations of 0.2 m mol/ℓ, 0.1%, 0.01%, 200 U/dℓ, 4,000 U/dℓ, 10 U/dℓ, and 0.5% respectively.

(Measuring method)

To 100 ml samples of serum ascorbic acid was added in an amount of 0, 5, 10, 20, 30, 60, 80, or 100 mg/dl and these solutions were used as samples.

To 20 µl of each sample solution were added 3 ml of the measuring reagent, and the mixture was incubated at 37°C for 5 minutes. Thereafter,the absorbance at a wavelength of 505 nm was measured with reference to a reagent blank as a control.

The concentration of the glucose is determined from a separately prepared calibration curve (Fig. 2). Measured results are shown in Table 8.

Comparative Example 2:

(Measuring reagent)

The same as the measuring reagent in Example 8 except that $CuSO_4 \cdot 5H_2O$ being excluded therefrom

(Measuring method)

Glucose was measured by using the same sample solutions as in Example 8 according to the measuring method in Example 8. Measured results are shown in Table 8.

Reference Example 2:

(Measuring reagent)

The same as the measuring reagent in Example 8 except that AOD is added to give a concentration of 100 U/dℓ instead of $CuSO_4 \cdot 5H_2O$

(Measuring method)

Glucose was measured by using the same sample solutions as in Example 8 according to the measuring method in Example 8. Measured results are shown in Table 8.

Table 8

| Added amount of ascorbic acid (mg/dℓ) | Glucose concentration (mg/dℓ) | | |
|---|---|---|---|
| | Example 8 | Comparative Example 2 | Reference Example 2 |
| 0 | 154 (100) | 154 (100) | 153 (100) |
| 5 | 155 (100.6) | 149 ( 96.8) | 154 (100.7) |
| 10 | 154 (100) | 137 ( 89.6) | 154 (100.7) |
| 20 | 152 ( 98.7) | 117 ( 76.0) | 152 ( 99.3) |
| 30 | 143 ( 92.9) | 101 ( 65.6) | 145 ( 94.8) |
| 60 | 100 ( 64.9) | 18 ( 11.7) | 95 ( 62.1) |
| 80 | 91 ( 59.1) | 3 ( 1.9) | 90 (58.8) |
| 100 | 84 ( 54.5) | 0 ( 0 ) | 81 (52.9) |
| Note: Figures in the parentheses mean recovery rates (%) of glucose. | | | |

As shown in Table 8, when up to 20 mg/dℓ of ascorbic acid are coexistent in Example 8 in which the copper ion, POD, phenol, and 4-AAP also are coexistent, no influence is observed upon the values of the glucose, while even when only 5 mg/dℓ of ascorbic acid are coexistent in Comparative Example 2, a negative influence appears.

As shown in Table 8, it is understood that the effect of removing the interference of ascorbic acid according to the method of the present invention is substantially equal to that of decomposing it with AOD.

Example 9: Measurement of free cholesterol (Two liquid method)

(Measuring reagent)

(1) First reagent liquid:

$CuSO_4 \cdot 5H_2O$, BSdipropM, POD, uricase and Triton X-100 were dissolved in 0.05 M phosphate buffer solution (pH = 7.0) to give concentrations of 0.01%, 0.1 m mol/ℓ, 500 U/dℓ, 30 U/dℓ, and 0.1% respectively.

(2) Second reagent solution:

Cholesterol oxidase and Triton X-100 were dissolved into 0.05 M phosphate buffer solution to give, concentrations of 20 U/dℓ, and 0.1% respectively.

(Measuring method)

To 100 ml samples of serum ascorbic acid was added to give concentrations of 10, 20, 30, 40, 50 or 100 mg/dl and these solutions were used as samples.

To 10 µl of each sample solution 1 ml of the first reagent solution was added and the mixture was incubated at 37°C for 5 minutes. Then, 2 ml of the second reagent solution were added, and the mixture was incubated at 37°C for 5 minutes. Thereafter, the absorbance at a wavelength of 620 nm was measured with reference to a reagent blank as a control. Measured results are shown in Table 9.

Comparative Example 3:

(Measuring reagent)

(1) First reagent solution:

The same as the first reagent solution in Example 9 except that $CuSO_4 \cdot 5H_2O$ is excluded therefrom.

(2) Second reagent solution:

The same as in Example 9

(Measuring method)

Free cholesterol was measured by using the same sample solutions as in Example 9 according to the measuring method in Example 9. Measured results are shown in Table 9.

Reference Example 3:

(Measuring reagent)

(1) First reagent solution:

AOD, BSdipropM, POD, uricase, and Triton X-100 were dissolved in 0.05 M phosphate buffer solution (pH = 7.0) to give concentrations of 30 U/dℓ, 0.1 m mol/ℓ, 500 U/dℓ, 30 U/dℓ and 0.1% respectively.

(2) Second reagent solution:

The same as in Example 9

(Measuring method)

Free cholesterol was measured by using the same sample soluti as in Example 9 according to the measuring method in Example 9. Measured results are shown in Table 9.

Example 10: Measurement of free cholesterol (one liquid method)

(Measuring reagent)

A mixture of the first reagent solution and the second reagent solution of Example 9 at a mixing ratio of 1 : 2 was used.

(Measuring method)

The same sample solutions as in Example 9 were used.

To 10 μℓ of each sample solution were added 3 mℓ of the measuring reagent and the mixture was incubated at 37°C for 10 minutes. Then, the absorbance at a wavelength of 620 nm was measured with reference to a reagent blank as a control. Measured results are shown in Table 9.

Comparative Example 4:

(Measuring reagent)

A mixture of the first reagent solution and the second reagent solution as in Comparative Example 3 at a mixing ratio of 1 : 2 was used.

(Measuring method)

Free cholesterol was measured by using the same sample solutions as in Example 9 according to the measuring method in Example 10. Measured results are shown in Table 9.

Reference Example 4:

(Measuring reagent)

A mixture of the first reagent solution and the second reagent solution as in Reference Example 3 at a mixing ratio of 1 : 2 was used.

(Measuring method)

Free cholesterol was measured by using the same sample solutions as in Example 9 according to the measuring method in Example 10. Measured results are shown in Table 9.

Table 9

| Absorbance (OD) / Added amount of ascorbic acid (mg/dℓ) | Two liquid method | | | One liquid method | | |
|---|---|---|---|---|---|---|
| | Example 9 ($Cu^{2+}$ contained) | Comparative Example 3 | Reference Example 3 (AOD contained) | Example 10 ($Cu^{2+}$ contained) | Comparative Example 4 | Reference Example 4 (AOD contained) |
| 0 | 0.568 | 0.559 | 0.571 | 0.560 | 0.563 | 0.570 |
| 10 | 0.569 | 0.478 | 0.568 | 0.481 | 0.451 | 0.501 |
| 20 | 0.568 | 0.376 | 0.561 | 0.414 | 0.330 | 0.426 |
| 30 | 0.568 | 0.202 | 0.573 | 0.384 | 0.165 | 0.412 |
| 40 | 0.561 | 0.078 | 0.562 | 0.351 | 0.051 | 0.370 |
| 50 | 0.567 | 0 | 0.554 | 0.329 | 0 | 0.363 |
| 100 | 0.562 | 0 | 0.536 | 0.313 | 0 | 0.342 |

As shown in Table 9, the effect of removing the interference of ascorbic acid according to the method of the present invention is equivalent to or much better than decomposing it with AOD with respect to the one liquid method and the two liquid method, and the influence of up to 100 mg/dl of ascorbic acid can be completely avoided in the case of the two liquid method.

Example 11: Measurement of free cholesterol (Two liquid method)

(Measuring reagent)

The same as in Example 9

(Measuring method)

To 100 ml samples of serum bilirubin was added to give concentrations of 0, 5, 10, 15 or 20 mg/dl and these solutions were used as samples.

To 10 µl of each sample solution 1 ml of the first reagent solution was added, and the mixture was incubated at 37°C for 5 minutes. Then, 2 ml of the second reagent solution were added, and the mixture was further incubated at 37°C for 5 minutes. Thereafter, the absorbance at a wavelength of 620 nm was measured with reference to a reagent blank as a control. Measured results are shown in Table 10.

Comparative Example 5:

(Measuring reagent)

The same as in Comparative Example 3

(Measuring method)

Free cholesterol was measured by using the same sample solutions as in Example 11 according to the measuring method in Example 11. Measured results are shown in Table 10.

Reference Example 5:

(Measuring reagent)

The same as in Reference Example 3

(Measuring method)

Free cholesterol was measured by using the same sample solutions as in Example 11 according to the measuring method in Example 11. Measured results are shown in Table 10.

Example 12: Measurement of free cholesterol (one liquid method)

(Measuring reagent)

The same as in Example 10

(Measuring method)

The same sample solutions as in Example 11 were used.

To 10 µℓ of each sample solution were added 3 mℓ of the measuring reagent and the mixture was incubated at 37°C for 10 minutes. Then, the absorbance at a wavelength of 620 nm was measured with reference to a reagent blank as a control. Measured results are shown in Table 10.

Comparative Example 6:

(Measuring reagent)

The same as in Comparative Example 4

(Measuring method)

Free cholesterol was measured by using the same sample solutions as in Example 11 according to the measuring method in Example 12. Measuring results are shown in Table 10.

Reference Example 6:

(Measuring reagent)

The same as in Comparative Example 4

(Measuring method)

Free cholesterol was measured by using the same sample solutions as in Example 11 according to the measuring method in Example 12. Measured results are shown in Table 10.

Table 10

| Added amount of bilirubin (mg/dl) / Absorbance (OD) | Two liquid method: Example 11 ($Cu^{2+}$ contained) | Two liquid method: Comparative Example 5 | Two liquid method: Reference Example 5 (AOD contained) | One liquid method: Example 12 ($Cu^{2+}$ contained) | One liquid method: Comparative Example 6 | One liquid method: Reference Example 6 (AOD contained) |
|---|---|---|---|---|---|---|
| 0 | 0.572 | 0.563 | 0.568 | 0.572 | 0.572 | 0.576 |
| 5 | 0.562 | 0.510 | 0.530 | 0.550 | 0.510 | 0.526 |
| 10 | 0.526 | 0.454 | 0.486 | 0.493 | 0.460 | 0.475 |
| 15 | 0.473 | 0.433 | 0.456 | 0.472 | 0.447 | 0.442 |
| 20 | 0.442 | 0.382 | 0.438 | 0.421 | 0.384 | 0.440 |

As shown in Table 10, the effect of removing the interference of bilirubin according to the method of the present invention is equivalent to or much better than decomposing it with AOD with respect to both the one liquid method and the two liquid method.

Example 13:

(Measuring reagent)

$CuSO_4 \cdot 5H_2O$, ABTS, POD, cholesterol oxidase, and Triton X-100 were dissolved in 0.05 M phosphate buffer solution (pH = 7.0) to give concentrations of 0.003%, 0.1%, 300 U/dl, 15 U/dl, and 0.1%.

(Measuring method)

Aqueous solutions containing 100 mg/dl of cholesterol and 0, 10, 20, 30, 40 or 50 mg/dl of ascorbic acid in 100 ml were used as sample solutions.

To 20 µl of each sample solution were added 3 ml of the measuring reagent, and the mixture was incubated at 37 °C for 10 minutes. Then, the absorbance at a wavelength of 660 nm was measured with reference to a reagent blank as a control. Measured results are shown in Table 11.

Comparative Example 7:

(Measuring reagent)

Same as the measuring reagent in Example 13, except that $CuSO_4 \cdot 5H_2O$ is excluded therefrom.

(Measuring method)

Free cholesterol was measured by using the same sample solutions as in Example 13 according to the measuring method in Example 13. Measured results are shown in Table 11.

Table 11

| Absorbance (OD) / Ascorbic acid concentration in the sample solution (mg/dℓ) | Example 13 | Comparative Example 7 |
|---|---|---|
| 0 | 0.216 (100) | 0.210 (100) |
| 10 | 0.174 ( 81) | 0.165 ( 79) |
| 20 | 0.135 ( 62) | 0.108 ( 51) |
| 30 | 0.116 ( 54) | 0.056 ( 27) |
| 40 | 0.107 ( 50) | 0 ( 0) |
| 50 | 0.087 ( 40) | 0 ( 0) |

Figures in the parentheses mean recovery rates (%) of cholesterol.

It is seen from Table 11 that the effect of removing the interference of ascorbic acid is obtained when the copper ion, POD and ABTS are coexistent.

INDUSTRIAL APPLICABILITY

The method according to the present invention can be used for effectively removing the adverse affect of ascorbic acid coexisting in a sample for the quantitative measurement of an intended component in the

sample which is examined by utilizing the oxidation-reduction reaction.

**Claims**

1. A method of decomposing ascorbic acid into dehydroascorbic acid and $H_2O$, which is characterized in that copper ion, peroxidase, and one or more compounds selected from the group consisting of the following (1) - (7):
    (1) 4-aminoantipyrine
    (2) 3-methyl-2-benzothiazolinonehydrazone
    (3) 2,2'-azinobis(3-ethylbenzothiazoline-6-sulfonic acid)
    (4) triphenyl methane type leuco compounds
    (5) phenol compounds
    (6) aniline compounds
    (7) naphthol compounds

    are reacted with ascorbic acid.

2. A method of decomposing ascorbic acid according to claim 1, wherein the copper ion is a divalent copper ion.

3. A method according to claim 1, wherein the ascorbic acid to be decomposed is coexisting in a sample to be examined.

4. A method according to claim 3, wherein the sample to be examined is a body fluid, and the ascorbic acid is decomposed in a reaction according to claim 1, whereby the interfering influence of the ascorbic acid is avoided in a reaction in which a component in the body fluid is quantatively measured by an oxidation-reduction (redox) reaction.

5. A method according to claim 4, wherein the oxidation-reduction reaction (redox reaction) is an enzyme reaction in which an oxidase acts upon a substrate to produce $H_2O_2$.

6. A method according to claim 5, wherein the substrate is selected from the group consisting of glucose, cholesterol, glycerol, glycerol phosphate, choline, acylCoA, pyruvic acid, uric acid, xanthine and lactic acid, and the oxidase acting upon the substrate is selected from the group consisting of glucose oxidase, cholesterol oxidase, glycerol oxidase, glycerol phosphate oxidase, choline oxidase, acylCoA oxidase, pyruvate oxidase, uricase, xanthine oxidase and lactate oxidase.

7. A method according to claim 6, wherein the component in the body fluid is quantatively measured by measuring a color developed through oxidation of an oxidizable coloring reagent in the presence of peroxidase and $H_2O_2$.

8. A method according to claim 5, wherein the decomposition of ascorbic acid in the body fluid sample and the redox reaction for quantatively measuring a component in the same sample is carried out in a single step and results in a colorific reaction.

9. A method according to claim 1, wherein the triphenyl methane type leuco compound is selected from the group consisting of leucomalachite green, leucocrystal violet, bis(p-diethyl-aminophenyl)-2-sulfophenyl methane, bis-(p-diethylaminophenyl)-4-sulfopropoxyphenyl methane sodium salt, and bis(p-diethylaminophenyl)-3,4-disulfopropoxyphenyl methane disodium salt.

10. A method according to claim 1, wherein the phenol compound is selected from phenol, p-chlorophenol, 2,4-dichlorophenol, p-bromophenol, o-chlorophenol, and m-chlorophenol.

11. A method according to claim 1, wherein the aniline compound is selected from the group consisting of aniline, N,N-dimethylaniline, N.N-diethylaniline, N,N-diethyl-m-toluidine, 3-methyl-N-ethyl-N-($\beta$-hydroxyethyl)-aniline, N-ethyl-N-(2-hydroxy-3-sulfopropyl)-m-toluidine, 3,5-dimethyl-N-ethyl-N-(2-hydroxy-3-sulfopropyl)aniline and 3,5-dimethoxy-N-ethyl-(2-hydroxy-3-sulfopropyl)aniline.

12. A method according to claim 1, wherein the naphthol compound is selected from the group consisting

of 1-naphthol, 1-naphthol-2-sulfonic acid, 1-naphthol-2-carboxylic acid, 1-naphthol-8-sulfonic acid, 1-naphthol-3-sulfonic acid, and 1-naphthol-5-sulfonic acid.

**13.** A method of quantitatively measuring a component in a body fluid sample, characterized in that the interfering influence of a reducing substance in the sample is avoided by reacting it with
a) a copper ion,
b) peroxidase, and
c) one or more compounds selected from the group of (1) to (7)
(1) 4-aminoantipyrine
(2) 3-methyl-2-benzothiazolinonehydrazone
(3) 2,2'-azinobis(3-ethylbenzothiazoline-6-sulfonic acid)
(4) triphenyl methane type leuco compound
(5) phenol compounds
(6) aniline compounds
(7) naphthol compounds
and by reacting the component in the body fluid sample with an oxidase to produce $H_2O_2$ which is then quantitatively measured in the presence of the reagents described under b) and c).

**14.** A quantitatively measuring method according to claim 13, wherein the reducing substance is ascorbic acid.

**15.** A quantitatively measuring method according to claim 13, wherein the reducing substance is bilirubin.

**Patentansprüche**

**1.** Eine Methode zum Aufspalten von Ascorbinsäure in Dehydroascorbinsäure und $H_2O$, die dadurch gekennzeichnet ist, daß ein Kupferion, Peroxidase und eine oder mehrere Verbindungen, ausgewählt aus der Gruppe, bestehend aus den folgenden Verbindungen (1) bis (7):
(1) 4-Aminoantipyrin
(2) 3-Methyl-2-benzothiazolinonhydrazon
(3) 2,2'-Azinobis(3-äthylbenzothiazolin-6-sulfonsäure)
(4) Leuko-Verbindungen vom Triphenylmethan-Typ
(5) Phenolverbindungen
(6) Anilinverbindungen
(7) Naphtholverbindungen
mit Ascorbinsäure umgesetzt werden.

**2.** Eine Methode zum Aufspalten von Ascorbinsäure nach Anspruch 1, bei welcher das Kupferion ein zweiwertiges Kupferion ist.

**3.** Eine Methode nach Anspruch 1, bei welcher die aufzuspaltende Ascorbinsäure in einer zu untersuchenden Probe vorliegt.

**4.** Eine Methode nach Anspruch 3, bei welcher die zu untersuchende Probe eine Körperflüssigkeit ist, und die Ascorbinsäure in einer Reaktion nach Anspruch 1 aufgespalten wird, wodurch der störende Einfluß der Ascorbinsäure bei einer Reaktion, in welcher eine Komponente der Körperflüssigkeit durch eine Redox-Reaktion quantitativ gemessen wird, vermieden wird.

**5.** Eine Methode nach Anspruch 4, bei welcher die Redox-Reaktion eine Enzym-Reaktion ist, in welcher eine Oxidase unter Bildung von $H_2O_2$ auf ein Substrat wirkt.

**6.** Eine Methode nach Anspruch 5, bei welcher das Substrat ausgewählt ist aus der Gruppe , bestehend aus Glukose, Cholesterin, Glycerin, Glycerinphosphat, Cholin, AcylCoA, Benztraubensäure, Harnsäure, Xanthin und Milchsäure, und die Oxidase, die auf das Substrat wirkt ausgewählt ist aus der Gruppe, bestehend aus Glukoseoxidase, Cholesterinoxidase, Glycerinoxidase, Glycerinphosphatoxidase, Cholinoxidase,AcylCoA-Oxidase, Pyruvatoxidase, Uricase, Xanthinoxidase und Laktatoxidase.

**7.** Eine Methode nach Anspruch 6, bei welcher die Komponente in der Körperflüssigkeit quantitativ

bestimmt wird durch Messen einer durch Oxidation eines oxidierbaren Färbereagens in Gegenwart von Peroxidase und $H_2O_2$ entwickelten Farbe.

**8.** Eine Methode nach Anspruch 5, bei welcher die Aufspaltung von Ascorbinsäure in der Körperflüssigkeitsprobe und die Redox-Reaktion zum quantitativen Messen einer Komponente in der gleichen Probe in einem einzigen Arbeitsgang durchgeführt werden und zu einer Färbungsreaktion führt.

**9.** Eine Methode nach Anspruch 1, bei welcher die Leukoverbindung vom Triphenylmethantyp ausgewählt ist aus der Gruppe, bestehend aus Leukomalachitgrün, Leukokristallviolett, Bis-(p-diäthyl-aminophenyl)-2-sulfophenylmethan, Bis-(p-diäthylaminophenyl)-4-sulfopropoxyphenylmethan-natriumsalz, und Bis(p-diäthylaminophenyl)-3,4-disulfopropoxyphenylmethan-dinatriumsalz.

**10.** Eine Methode nach Anspruch 1, bei welcher die Phenolverbindung ausgewählt ist aus Phenol, p-Chlorphenol, 2,4-Dichlorphenol, p-Bromphenol, o-Chlorphenol, und m-Chlorphenol.

**11.** Eine Methode nach Anspruch 1, in welcher die Anilinverbindung ausgewählt ist aus der Gruppe, bestehend aus Anilin, N,N-Dimethylanilin, N,N-Diäthylanilin, N,N-Diäthyl-m-toluidin, 3-Methyl-N-äthyl-N-($\beta$-hydroxyäthyl)-anilin, N-Äthyl-N-(2-hydroxy-3-sulfopropyl)-m-toluidin, 3,5-Dimethyl-N-äthyl-N-(2-hydroxy-3-sulfopropyl)anilin und 3,5-Dimethoxy-N-äthyl-(2-hydroxy-3-sulfopropyl)anilin.

**12.** Eine Methode nach Anspruch 1, in welcher die Naphtholverbindung ausgewählt ist aus der Gruppe, bestehend aus 1-Naphthol, 1-Naphthol-2-sulfonsäure, 1-Naphthol-2-carbonsäure, 1-Naphthol-8-sulfonsäure, 1-Naphthol-3-sulfonsäure, und 1-Naphthol-5-sulfonsäure.

**13.** Eine Methode zum quantitativen Messen einer Komponente in einer Körperflüssigkeitsprobe, dadurch gekennzeichnet, daß der störende Einfluß einer reduzierend wirkenden Substanz in der Probe durch Umsetzung mit
a) einem Kupferion
b) Peroxidase, und
c) einer oder mehreren Verbindungen, ausgewählt aus der Gruppe der Verbindungen von (1) bis (7)
(1) 4-Aminoantipyrin
(2) 3-Methyl-2-benzothiazolinonhydrazon
(3) 2,2'-Azinobis(3-äthylbenzothiazolin-6-sulfonsäure)
(4) Leuko-Verbindungen vom Triphenylmethan-Typ
(5) Phenolverbindungen
(6) Anilinverbindungen
(7) Naphtholverbindungen
vermieden wird und daß die Komponente in der Körperflüssigkeitsprobe mit einer Oxidase unter Bildung von $H_2O_2$ umgesetzt wird, welches dann in Gegenwart der unter b) und c) genannten Reagentien quantitativ bestimmt wird.

**14.** Eine Methode zum quantitativen Messen nach Anspruch 13, in welcher die reduzierend wirkende Substanz Ascorbinsäure ist.

**15.** Eine Methode zum quantitativen Messen nach Anspruch 13, in welcher die reduzierend wirkende Substanz Bilirubin ist.

**Revendications**

**1.** Procédé pour décomposer l'acide ascorbique en de l'acide déshydrosascorbique et $H_2O$, qui est caractérisé en ce que l'on fait réagir avec l'acide ascorbique l'ion cuivre, de la peroxydase et un ou plusieurs composés choisis dans l'ensemble consistant en les composés (1) à (7) suivants :
(1) la 4-aminoantipyrine
(2) la 3-méthyl-2-benzothiazolinonehydrazone
(3) le 2,2'-azinobis(acide 3-éthylbenzothiazoline-6-sulfonique)
(4) des leuco dérivés de composés du type du triphénylméthane
(5) des composés phénoliques
(6) des anilines

(7) des naphtols.

**2.** Procédé pour décomposer l'acide ascorbique selon la revendication 1, dans lequel l'ion cuivre est un ion cuivre divalent.

**3.** Procédé selon la revendication 1, dans lequel l'acide ascorbique à décomposer est également présent dans un échantillon à examiner.

**4.** Procédé selon la revendication 3, dans lequel l'échantillon à examiner est un fluide corporel, et l'acide ascorbique est décomposé dans une réaction selon la revendication 1, de sorte que l'on évite l'influence gênante de l'acide ascorbique dans une réaction dans laquelle on mesure quantitativement, par une réaction d'oxydation-réduction (réaction d'oxydo-réduction ou de type redox), un constituant présent dans le fluide corporel.

**5.** Procédé selon la revendication 4, dans lequel la réaction d'oxydation-réduction (réaction de type redox) est une réaction enzymatique dans laquelle une oxydase agit sur un substrat pour produire $H_2O_2$.

**6.** Procédé selon la revendication 5, dans lequel le substrat est choisi dans l'ensemble consistant en du glucose, du cholestérol, du glycérol, du phosphate de glycérol, de la choline, de l'acylCoA, de l'acide pyruvique, de l'acide urique, de la xanthine et de l'acide lactique, et l'oxydase agissant sur le substrat est choisie dans l'ensemble consistant en de la glucose oxydase, de la cholestérol oxydase, de la glycérol oxydase, de la (phosphate de glycérol)-oxydase, de la choline oxydase, de l'acylCoA oxydase, de la pyruvate oxydase, de l'uricase, de la xanthine oxydase et de la lactase oxydase.

**7.** Procédé selon la revendication 6, dans lequel on mesure quantitativement le constituant présent dans le fluide corporel en mesurant une couleur développée par suite de l'oxydation d'un réactif colorant oxydable en présence de peroxydase et de $H_2O_2$.

**8.** Procédé selon la revendication 5, dans lequel on effectue en une seule étape la décomposition de l'acide ascorbique présent dans l'échantillon de fluide corporel et la réaction reédox pour une mesure quantitative d'un constituant du même échantillon, ce qui aboutit à une réaction colorée.

**9.** Procédé selon la revendication 1, dans lequel on choisit le leuco dérivé du composé de type triphénylméthane dans l'ensemble consistant en du leuco (vert malachite), du leuco (violet cristallisé), du bis(p-diéthyl-aminophényl)-2-sulfophénylméthane, du bis(p-diéthylaminophényl)-4-sulfopropoxyphénylméthane, sel de sodium et le sel disodique du bis(p-diéthylaminophényl)-3,4-disulfopropoxyphénylméthane.

**10.** Procédé selon la revendication 1, dans lequel le composé phénolique est choisi parmi le phénol, le p-chlorophénol, le 2,4-dichlorophénol, le p-bromophénol, l'o-chlorophénol et le m-chlorophénol.

**11.** Procédé selon la revendication 1, dans lequel l'aniline est choisie dans l'ensemble consistant en l'aniline, la N,N-diméthylaniline, la N,N-diéthylaniline, la N,N-diéthyl-m-toluidine, la 3-méthyl-N-éthyl-N-($\beta$-hydroxyéthyl)-aniline, la N-éthyl-N-(2-hydroxy-3-sulfopropyl)-m-toluidine, la 3,5-diméthyl-N-éthyl-N-(2-hydroxy-3-sulfopropyl)aniline et la 3,5-diméthyoxy-N-éthyl-(2-hydroxy-3-sulfopropyl)aniline.

**12.** Procédé selon la revendication 1, dans lequel le naphtol est choisi dans l'ensemble consistant en le 1-naphtol, l'acide 1-naphtol-2-sulfonique, l'acide 1-naphtol-2-carboxylique, l'acide 1-naphtol-8-sulfonique, l'acide 1-naphtol-3-sulfonique et l'acide 1-naphtol-5-sulfonique.

**13.** Procédé de mesure quantitative d'un constituant présent dans un échantillon de fluide corporel, procédé caractérisé en ce que l'on évite l'influence gênante exercée par une substance réductrice présente dans l'échantillon en la faisant réagir avec :
(a) un ion cuivre
(b) une peroxydase, et
(c) un ou plusieurs composés choisis dans l'ensemble des composés (1) à (7) :
(1) la 4-aminoantipyrine
(2) la 3-méthyl-2-benzothiazolinonehydrazone

(3) le 2,2'-azinobis(acide 3-éthylbenzothiazoline-6-sulfonique)
(4) un leuco dérivé de composés de type triphénylméthane
(5) des composés phénoliques
(6) des anilines
(7) des naphtols

et en faisant réagir le constituant présent dans l'échantillon de fluide corporel avec une oxydase pour produire $H_2O_2$, que l'on mesure ensuite quantitativement en présence des réactifs décrits en (b) et en (c).

**14.** Procédé de mesure quantitative selon la revendication 13, dans lequel la substance réductrice est l'acide ascorbique.

**15.** Procédé de mesure quantitative selon la revendication 13, dans lequel la substance réductrice est la bilirudine.

FIG.1

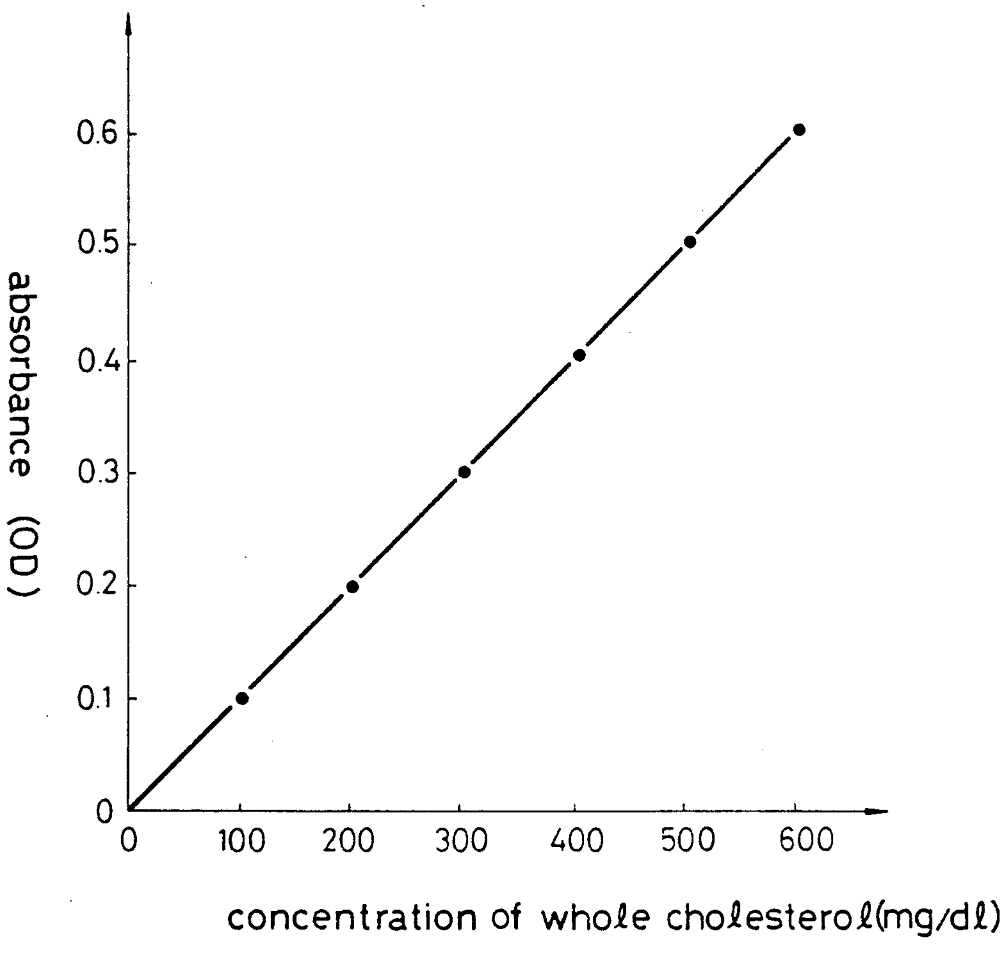
absorbance (OD)
0.6
0.5
0.4
0.3
0.2
0.1
0
0
100
200
300
400
500
600
concentration of whole cholesterol(mg/dl)

FIG. 2

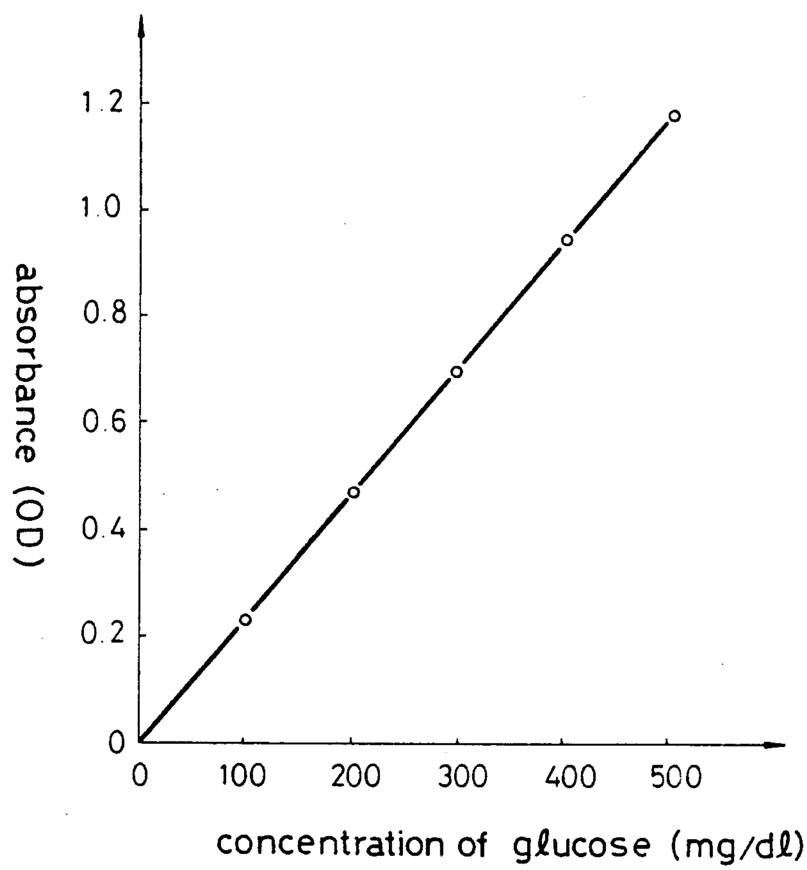
absorbance (OD)
1.2
1.0
0.8
0.6
0.4
0.2
0
0
100
200
300
400
500
concentration of glucose (mg/dl)